# EUROPEAN PATENT SPECIFICATION

(11) **EP 4 285 732 B1**
(45) Date of publication and mention of the grant of the patent: **12.11.2025**
(21) Application number: 23175926.7
(22) Date of filing: 30.05.2023
(51) Int. Cl.: A23F 3/16, A23L 33/105, A23L 2/62, A23L 2/52

(54) **COMPOSITION FOR REDUCING THE ABSORPTION OF MICROPLASTICS**
ZUSAMMENSETZUNG ZUR VERRINGERUNG DER ABSORPTION VON MIKROKUNSTSTOFFEN
COMPOSITION POUR RÉDUIRE L'ABSORPTION DE MICROPLASTIQUES

(30) Priority: 30.05.2022 KR 20220066326
(43) Date of publication of application: 06.12.2023
(73) Proprietor: Amorepacific Corporation, Seoul 04386 (KR)
(72) Inventor: JEONG, Hyun Woo, 17074 Gyeonggi-do (KR); CHUNG, Jinoh, 17074 Gyeonggi-do (KR); KIM, Wanki, 17074 Gyeonggi-do (KR); ROH, Jong Hwa, 17074 Gyeonggi-do (KR)
(74) Representative: Germain Maureau

(56) References cited:
- YAN JINGQI ET AL: "Green tea catechins prevent obesity through modulation of peroxisome proliferator-activated receptors", SCIENCE CHINA LIFE SCIENCES, ZHONGGUO KEXUE ZAZHISHE, CHINA, vol. 56, no. 9, 12 July 2013 (2013-07-12), pages 804 - 810, XP035710812, ISSN: 1674-7305, [retrieved on 20130712], DOI: 10.1007/S11427-013-4512-2
- RANA M. JAMOUS ET AL: "Antiobesity and Antioxidant Potentials of Selected Palestinian Medicinal Plants", EVIDENCE-BASED COMPLEMENTARY AND ALTERNATIVE MEDICINE, vol. 2018, 13 June 2018 (2018-06-13), US, pages 1 - 21, XP055734503, ISSN: 1741-427X, DOI: 10.1155/2018/8426752
- ZWIERELLO WOJCIECH ET AL: "The influence of polyphenols on metabolic disorders caused by compounds released from plastics - Review", CHEMOSPHERE, PERGAMON PRESS, OXFORD, GB, vol. 240, 20 September 2019 (2019-09-20), XP085888496, ISSN: 0045-6535, [retrieved on 20190920], DOI: 10.1016/J.CHEMOSPHERE.2019.124901
- CHENG WEI ET AL: "Polystyrene microplastics induce hepatotoxicity and disrupt lipid metabolism in the liver organoids", SCIENCE OF THE TOTAL ENVIRONMENT, ELSEVIER, AMSTERDAM, NL, vol. 806, 15 September 2021 (2021-09-15), XP086872168, ISSN: 0048-9697, [retrieved on 20210915], DOI: 10.1016/J.SCITOTENV.2021.150328

## Description

### BACKGROUND OF THE INVENTION

### Field of the Invention

The disclosure relates to a composition for reducing the absorption of microplastics.

### Description of the Related Art

In general, microplastics refer to synthetic polymer compounds with a size of 5 mm or less, and are classified into primary microplastics and secondary microplastics based on origin. Primary microplastics refer to plastic particles produced for an intended purpose, e.g., resin pellets, scrubs in facial wash or toothpaste, industrial abrasives, or the like. Secondary microplastics refer to micronized plastic fragments that are decomposed or pulverized into small sizes in the process of using plastic products or due to corrosion by ultraviolet rays or various collisions of discarded plastic waste. These microplastics are not filtered out in a sewage treatment plant and flow into the sea through streams and rivers.

Microplastics can adsorb, transport, and release not only toxic persistent organic pollutants such as polychlorinated biphenyls(PCBs), dioxins, and dichlorodiphenyl-trichloroethane(DDT), but also endocrine disruptors such as phthalates and bisphenols. Thus, microplastics are also called deadly bites of grain, which have serious adverse effects on rivers, marine environments and various ecosystems. After continuously accumulated in the body of an organism along the food chain, the microplastics are finally consumed by humans in the form of food, and the microplastics can also be included in tap water (drinking water) that has gone through a purification process. Through this process, when a large amount of microplastics is accumulated in the body as microplastics are consumed for a long time, the microplastics become a factor of carcinogenesis or cause health damage by environmental hormone.

Cheng Wei et al: "Polystyrene microplastics induce hepatotoxicity and disrupt lipid metabolism in the liver organoids", Science of the Total Environment, Elsevier, Amsterdam, NL, vol. 806, 15 September 2021, discloses that microplastic particles can affect the human body by causing hepatotoxicity and interfering with lipid metabolism.

Zwierello Wojciech et al: "The influence of polyphenols on metabolic disorders caused by compound released from plastics - Review", Chemosphere, Pergamon Press, Oxford, GB, vol. 240, 20 September 2019, discloses that epigallocatechin gallate (EGCG) is a polyphenol belonging to the catechin family found in large quantities in tea, and that it can inhibit the pro-inflammatory effects caused by plastics such as polychlorinated biphenyls (PCBs).

Rana M. Jamous et al: "Antiobesity and Antioxidant Potentials of Selected Palestinian Medicinal Plants", Evidence-based Complementary and Alternative Medicine, vol. 2018, 13 June 2018, discloses the anti-obesity effect of green tea extract, especially various polyphenols (EGCG, ECG, EGC) isolated from green tea leaves, and the efficacy of improving systemic inflammation, hyperglycemia, and insulin resistance.

Yan Jingqi et al: "Green tea catechins prevent obesity through modulation of peroxisome proliferator-activated receptors", Science China Life Sciences, Zhongguo Kexue Zazhishe, China, vol. 56, no. 9, 12 July 2013, pages 804-810, discloses that administration of green tea extract or green tea catechins has an anti-obesity effect because it reduces total body lipids and body weight and increases the expression of genes involved in fatty acid oxidation. It also discloses that it can lower the risk of related diseases (hyperlipidemia, metabolic syndrome, etc.).

### SUMMARY OF THE INVENTION

In one aspect, the present invention provides a composition for use in preventing, alleviating or treating lipid metabolism disorder by reducing absorption of microplastics into the body, comprising a green tea extract as an active ingredient.

Another aspect of the invention is a non-therapeutic use of a food composition for reducing absorption of microplastics into the body, comprising a green tea extract as an active ingredient. In an exemplary embodiment, the green tea extract may be an extract of water, an organic solvent, or a mixed solvent thereof.

In an exemplary embodiment, the green tea extract may comprise 30 to 45 % by weight of catechin based on a total weight of the green tea extract.

In an exemplary embodiment, the green tea extract may be administered at a dosage of 1 to 200 mg/kg/day.

In one exemplary embodiment, the composition may aggregate the microplastics.

In one exemplary embodiment, the composition may inhibit inflammation caused by absorption of the microplastics.

In one exemplary embodiment, the composition may inhibit oxidative stress in a cell caused by the microplastics.

In one exemplary embodiment, the composition may prevent, alleviate or treat lipid metabolism disorder in a cell caused by the microplastics.

In one exemplary embodiment, the composition may be administered orally.

In an exemplary embodiment, the composition may be a pharmaceutical composition.

### BRIEF DESCRIPTION OF THE DRAWINGS

FIG. 1 is a graph showing an expression level of genes related to an inflammatory response of macrophages by microplastics.
FIG. 2 is a graph showing a degree of secretion of inflammatory factors of macrophages by microplastics.
FIG. 3 is a graph showing a degree of production of reactive oxygen species and nitrogen oxides in macrophages by microplastics.
FIG. 4 is a graph showing an expression level of genes related to an inflammatory response of hepatocytes by microplastics.
FIG. 5 is a graph showing a degree of production of reactive oxygen species, nitrogen oxides, and lipid peroxides in hepatocytes by microplastics.
FIG. 6 is a graph showing expression levels of SREBP1c, ACC and FAS genes of hepatocytes by microplastics.
FIG. 7 is a graph showing expression levels of ACO, CPT and PPARα genes of hepatocytes by microplastics.
FIG. 8 shows a reduction of a number of mitochondria in hepatocytes by microplastics.
FIG. 9 shows lipid accumulation in hepatocytes by microplastics.
FIG. 10 shows a degree of microplastic aggregation by a green tea extract according to one embodiment of the disclosure.
FIG. 11 shows a comparison of microplastic aggregation effects of a green tea extract according to an embodiment of the disclosure and a coffee extract.
FIG. 12 shows an amount of microplastics that migrate down a transwell in a permeation experiment using intestinal cells according to an embodiment of the disclosure.
FIG. 13 is a graph showing an effect of inhibiting inflammation caused by microplastics of a green tea extract according to one embodiment of the disclosure.
FIG. 14 is a graph showing expression levels of genes related to fatty acid oxidation by a green tea extract according to one embodiment of the disclosure.
FIG. 15 is a graph showing an effect of inhibiting an expression of genes related to inflammation caused by microplastics of a green tea extract according to an embodiment of the disclosure.
FIG. 16 is a graph showing an effect of inhibiting oxidative stress caused by microplastics of a green tea extract according to an embodiment of the disclosure.
FIG. 17 is a graph showing that a green tea extract according to one embodiment of the disclosure inhibits an expression of genes related to inflammation caused by microplastics in hepatocytes.
FIG. 18 is a graph showing that a green tea extract according to an embodiment of the disclosure inhibits oxidative stress caused by microplastics in hepatocytes.
FIG. 19 is a graph showing that a green tea extract according to one embodiment of the disclosure promotes an expression of lipid oxidation genes in hepatocytes.
FIG. 20 shows that a green tea extract according to one embodiment of the disclosure improves mitochondria reduced by microplastics in hepatocytes.
FIG. 21 shows that a green tea extract according to one embodiment of the disclosure inhibits lipid accumulation caused by microplastics in hepatic cells.

### DETAILED DESCRIPTION OF THE INVENTION

Although the terms used in the specification are selected from generally known and used terms while considering functions of the disclosure, they may vary according to intention or customs of those skilled in the art or emergence of new technology. In certain cases, some of the terms may have been selected by the applicant at his or her discretion, and in such cases the detailed meanings thereof will be described in relevant parts of the description herein. Thus, the terms used in this specification should be interpreted based on the substantial meanings of the terms and the whole content of this specification rather than their simple names or meanings.

Not only technical or scientific terms but also all terms used herein shall have the same meaning as commonly understood by one of ordinary skill in the art to which this invention belongs, unless otherwise defined. Terms that are generally understood should be interpreted as having a meaning that is consistent with their meaning in the context of the relevant art and should not be interpreted in an idealized or overly formal sense unless expressly so defined herein.

Numerical ranges include the numbers defined in the disclosure. All maximum numerical limitations defined in this specification include all lower numerical limitations, as if such limitations were expressly written. All minimum numerical limitations defined in this specification include all higher numerical limitations as if such higher numerical limitations were expressly written. All numerical limitations defined in this specification include all preferred numerical ranges within their broader numerical ranges as if such narrower numerical limitations were expressly written.

As used in the specification, the terms "comprising," "having," and "containing" are inclusive or open-ended and do not exclude features or method steps that are not further mentioned. The term "or a combination thereof" as used herein refers to all permutations and combinations of the plurality of items listed prior to the term. For example, "A, B, C, or combinations thereof" is intended to include at least one of: A, B, C, AB, AC, BC, or ABC and, if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth.

Hereinafter, exemplary embodiments of the disclosure are described in detail.

In one aspect, the disclosure provides a composition for reducing absorption of microplastics into the body, comprising a green tea extract as an active ingredient.

In the specification, "green tea" refers to tea *(Camellia sinensis),* which is an evergreen shrub belonging to the tea family, and can be used without limitation on its type and cultivation days, and the tea may include one or more selected from the group consisting of leaves, flowers, stems, fruits, and roots of tea tree. In addition, in the specification, green tea may be used without limitation in its parts (leaves, flowers, stems, fruits, roots, etc.), and for example, tea leaves may be used.

In the specification, "green tea extract" includes an extract extracted from *Camellia sinensis,* which belongs to the tea family, regardless of the extraction method, extraction solvent, extracted ingredient, or form of extract, an extract extracted from tea leaves inoculated with *Bacillus subtilis* spp. and fermented, an extract extracted from naturally fermented tea leaves, or the like, and includes a fraction obtained by fractionating the extracted extract with a specific solvent. The tea may include one or more selected from the group consisting of leaves, flowers, stems, fruits, and roots, and may preferably be leaves. Further, the form of extract may be preferably in a powder form. The extraction or fractionation may be performed using water, an organic solvent, or a mixed solvent thereof. The organic solvent may be an alcohol, isopropanol, acetone, hexane, ethyl acetate, carbon dioxide, or a mixed solvent of two or more thereof, and an active ingredient of green tea may be extracted or fractionated at room temperature or by heating under the condition that the active ingredient is not destroyed or is minimized. The alcohol may be a C1 to C6 alcohol. It is possible to use a method such as cold precipitation extraction, ultrasonic extraction, reflux cooling extraction, and hot-water extraction, and preferably, the active ingredient may be extracted or fractionated by cold precipitation or heating and filtered, and then the filtrate may be concentrated under reduced pressure to obtain the green tea extract.

In the specification, the "active ingredient" refers to an ingredient that alone exhibits the desired activity or that can exhibit the activity in combination with a carrier having no activity by itself.

In the specification, the "microplastics" refer to synthetic high-molecular compounds with a diameter of 5 mm or less that are intentionally manufactured or micronized by fragmenting existing plastic products.

In one embodiment, the green tea extract may be an extract of water, an organic solvent, or a mixed solvent thereof. The water may include distilled water or purified water, and the organic solvent may include one or more selected from the group consisting of alcohol, isopropanol, acetone hexane, ethyl acetate, and carbon dioxide. The alcohol may be a C1-C6 alcohol.

In one embodiment, the green tea extract may include 30 to 45 % by weight of catechin based on the total weight of the green tea extract. For example, the green tea extract may include 30 % by weight or more, 30.5 % by weight or more, 31 % by weight or more, 31.5 % by weight or more, 32 % by weight or more, 32.5 % by weight or more, 33 % by weight or more, 33.5 % by weight or more, 34 % by weight or more, 34.5 % by weight or more, 35 % by weight or more, 45 % by weight or less, 44.5 % by weight or less, 44 % by weight or less, 43.5 % by weight or less, 43 % by weight or less, 42.5 % by weight or less, 42 % by weight or less, 41.5 % by weight or less, 41 % by weight or less, 40.5 % by weight or less, or 40 % by weight or less of catechin based on the total weight of the green tea extract.

In one embodiment, the composition may include 0.1 to 70 % by weight of green tea extract based on the total weight of the composition. If the content is less than 0.1 % by weight, the effect of reducing the absorption of microplastics may be insignificant, and if the content is greater than 70 % by weight, the efficacy of reducing the absorption of microplastics may be reduced. For example, the composition may include 0.1 % by weight or more, 0.2 % by weight or more, 0.3 % by weight or more, 0.4 % by weight or more, 0.5 % by weight or more, 0.6 % by weight or more, 0.7 % by weight or more, 0.8 % by weight or more, 0.9 % by weight or more, 1 % by weight or more, 2 % by weight or more, 3 % by weight or more, 4 % by weight or more, 5 % by weight or more, 6 % by weight or more, 7 % by weight or more, 8 % by weight or more, 9 % by weight or more, 10 % by weight or more, 11 % by weight or more, 12 % by weight or more, 13 % by weight or more, 14 % by weight or more, 15 % by weight or more, 16 % by weight or more, 17 % by weight or more, 18 % by weight or more, 19 % by weight or more, 20 % by weight or more, 21 % by weight or more, 22 % by weight or more, 23 % by weight or more, 24 % by weight or more, 25 % by weight or more, 26 % by weight or more, 27 % by weight or more, 28 % by weight or more, 29 % by weight or more, 30 % by weight or more, 70 % by weight or less, 69.9 % by weight or less, 69.8 % by weight or less, 69.7 % by weight or less, 69.5 % by weight or less, 69 % by weight or less, 68.5 % by weight or less, 68 % by weight or less, 67.5 % by weight or less, 67 % by weight or less, 66.5 % by weight or less, 66 % by weight or less, 65.5 % by weight or less, 65 % by weight or less, 64.5 % by weight or less, 64 % by weight or less, 63.5 % by weight or less, 63 % by weight or less, 62.5 % by weight or less, 62 % by weight or less, 61.5 % by weight or less, 61 % by weight or less, 60.5 % by weight or less, 60 % by weight or less, 59 % by weight or less, 58 % by weight or less, 57 % by weight or less, 56 % by weight or less, 55 % by weight or less, 54 % by weight or less, 53 % by weight or less, 52 % by weight or less, 51 % by weight or less, 50 % by weight or less, 49 % by weight or less, 48 % by weight or less, 47 % by weight or less, 46 % by weight or less, 45 % by weight or less, 44 % by weight or less, 43 % by weight or less, 42 % by weight or less, 41 % by weight or less, 40 % by weight or less, 39 % by weight or less, 38 % by weight or less, 37 % by weight or less, 36 % by weight or less, 35 % by weight or less, 34 % by weight or less, 33 % by weight or less, 32 % by weight or less, 31 % by weight or less, 30 % by weight or less, 29 % by weight or less, 28 % by weight or less, 27 % by weight or less, 26 % by weight or less, 25 % by weight or less, 24 % by weight or less, 23 % by weight or less, 22 % by weight or less, 21 % by weight or less, 20 % by weight or less, 19 % by weight or less, 18 % by weight or less, 17 % by weight or less, 16 % by weight or less, 15 % by weight or less, 14 % by weight or less, 13 % by weight or less, 12 % by weight or less, 11 % by weight or less, or 10 % by weight or less of the green tea extract, based on the total weight of the composition.

In one embodiment, the green tea extract may be administered at a dosage of 1 to 200 mg/kg/day. If the dosage of the green tea extract is less than 1 mg/kg/day, the effect of reducing the absorption of microplastics may be insignificant, and if the dosage exceeds 200 mg/kg/day, the efficiency of reducing the absorption of microplastics may be reduced. Particularly, the dosage of the green tea extract may be 1 mg/kg/day or more, 1.1 mg/kg/day or more, 1.2 mg/kg/day or more, 1.3 mg/kg/day or more, 1.4 mg/kg/day or more, 1.5 mg/kg/day or more, 1.6 mg/kg/day or more, 1.7 mg/kg/day or more, 1.8 mg/kg/day or more, 1.9 mg/kg/day or more, 2 mg/kg/day or more, 2.5 mg/kg/day or more, 3 mg/kg/day or more, 4 mg/kg/day or more, 5 mg/kg/day or more, 6 mg/kg/ day or more, 7 mg/kg/day or more, 8 mg/kg/day or more, 9 mg/kg/day or more, 10 mg/kg/day or more, 11 mg/kg/day or more, 12 mg/kg/day or more, 13 mg/kg/day or more, 14 mg/kg/day or more, 15 mg/kg/day or more, 16 mg/kg/day or more, 17 mg/kg/day or more, 18 mg/kg/day or more, 19 mg/kg/day or more, 20 mg/kg/day or more, 21 mg/kg/day or more, 22 mg/kg/day or more, 23 mg/kg/day or more, 24 mg/kg/day or more, 25 mg/kg/day or more, 26 mg/kg/day or more, 27 mg/kg/day or more, 28 mg/kg/day or more, 29 mg/kg/day or more, 30 mg/kg/day or more, 31 mg/kg/day or more, 32 mg/kg/day or more, 33 mg/kg/day or more, 34 mg/kg/day or more, 35 mg/kg/day or more, 36 mg/kg/day or more, 37 mg/kg/day or more, 38 mg/kg/day or more, 39 mg/kg/day or more, 40 mg/kg/day or more, 41 mg/kg/day or more, 42 mg/kg/day or more, 43 mg/kg/day or more, 44 mg/kg/day or more, 45 mg/kg/day or more, 46 mg/kg/day or more, 47 mg/kg/day or more, 48 mg/kg/day or more, 49 mg/kg/day or more, 50 mg/kg/day or more, 200 mg/kg/day or less, 199 mg/kg/day or less, 198 mg/kg/day or less, 197 mg/kg/day or less, 196 mg/kg/day or less, 195 mg/kg/day or less, 194 mg/kg/day or less, 193 mg/kg/day or less, 192 mg/kg/day or less, 191 mg/kg/day or less, 190 mg/kg/day or less, 189 mg/kg/day or less, 188 mg/kg/day or less, 187 mg/kg/day or less, 186 mg/kg/day or less, 185 mg/kg/day or less, 184 mg/kg/day or less, 183 mg/kg/day or less, 182 mg/kg/day or less, 181 mg/kg/day or less, 180 mg/kg/day or less, 179 mg/kg/day or less, 178 mg/kg/day or less, 177 mg/kg/day or less, 176 mg/kg/day or less, 175 mg/kg/day or less, 174 mg/kg/day or less, 173 mg/kg/day or less, 172 mg/kg/day or less, 171 mg/kg/day or less, 170 mg/kg/day or less, 169 mg/kg/day or less, 168 mg/kg/day or less, 167 mg/kg/day or less, 166 mg/kg/day or less, 165 mg/kg/day or less, 164 mg/kg/day or less, 163 mg/kg/day or less, 162 mg/kg/day or less, 161 mg/kg/day or less, 160 mg/kg/day or less, 159 mg/kg/day or less, 158 mg/kg/day or less, 157 mg/kg/day or less, 156 mg/kg/day or less, 155 mg/kg/day or less, 154 mg/kg/day or less, 153 mg/kg/day or less, 152 mg/kg/day or less, 151 mg/kg/day or less, or 150 mg/kg/day or less.

**In** one embodiment, the microplastic may be, for example, a plastic such as polystyrene, polypropylene, polyethylene, polyamide(PA), acrylonitrile-butadienestyrene(ABS), polytetrafluoroethylene(PTFE), cellulose acetate(CA), polycarbonate(PC), polymethyl methacrylate(PMMA), polyvinyl chloride(PVC), polyethylene terephthalate(PET), acrylic, melamine resin, polyurethane(PU).

**In** one embodiment, the composition may aggregate microplastics. As the size of a material increases, penetration of microplastics through cells or periplasmic space becomes difficult. The composition may reduce the absorption of microplastics into the body by aggregating the ingested microplastics, making it difficult for the microplastics to pass through cells or periplasmic space, and excreting them out of the body through excretion.

**In** one exemplary embodiment, the composition may inhibit inflammation caused by absorption of the microplastics.

**In** one exemplary embodiment, the composition may inhibit oxidative stress in a cell caused by the microplastics.

**In** one exemplary embodiment, the composition may prevent, alleviate or treat lipid metabolism disorder in a cell caused by the microplastics.

**In** one exemplary embodiment, the composition may be administered orally.

**In** one aspect of the invention the composition may be a food composition. The formulation of the food composition may be made in, for example, tablets, granules, pills, powders, solutions such as drinks, caramels, gels, bars, tea bags, or the like. **In** the food composition of the respective formulations, in addition to the active ingredient, ingredients typically used in the pertinent field can be suitably selected and blended by a person skilled in the art without any difficulty, depending on the formulation or purpose of use, and when this composition is applied together with other raw material, a synergistic effect would be generated.

The food composition according to one embodiment may include several nutritional supplements, vitamins, minerals (electrolyte), flavoring agents such as synthetic flavoring agents and natural flavoring agents, colorants and enhancers (cheese, chocolate, etc.), pectic acids and salts thereof, alginic acids and salts thereof, organic acids, protective colloid thickeners, pH regulators, stabilizers, preservatives, glycerin, alcohol, carbonation used for carbonated beverages, and the like. In addition, the food compositions according to one embodiment may include natural fruit juices and fruit pulps for the preparation of fruit juice drink and vegetable drink. Such ingredients may be used independently or in combination. The ratio of such additives is not important, but the additives are generally comprised in an amount ranging from 0 to about 50 parts by weight per the 100 parts by weight of the composition according to one embodiment.

In one embodiment, the composition may be a pharmaceutical composition. The pharmaceutical composition may be administered orally, parenterally, rectally, topically, transdermally, intravenously, intramuscularly, intraperitoneally, subcutaneously, or the like. The formulation for the oral administration may be tablets, pills, soft and hard capsules, granules, powders, fine granules, solutions, emulsifying agents or pellets. The formulation for the parenteral administration may be solutions, suspensions, liquids, gels, injections, drops, suppository, patches or sprays. The formulations can be easily prepared according to a commonly employed method in the pertinent field, and may further comprise surfactants, excipients, hydrating agent, emulsifiers, suspensions, salts and/or buffers for osmotic pressure control, colorants, spices, stabilizers, antimicrobial agents, preservatives, or other commercial auxiliaries.

Hereinafter, the disclosure will be explained in more detail with reference to examples. These examples are only for illustrating the disclosure.

### [Example 1] Preparation of a green tea extract

After drying green tea leaves (*Camellia sinensis,* Jeju O'Sulloc Farm), 50 % (v/v) alcohol in an amount equivalent to 20 times the weight of the dried green tea leaves was added to the dried green tea leaves, extracted at 60 °C for 2 hours, and freeze-dried to prepare a green tea extract. The content of catechin in the green tea extract thus prepared was measured using high performance liquid chromatography(HPLC), and the results were shown in Table 1 below.

**[Table 1]**

| | Content (wt%) |
|---|---|
| Caffeine (CAF) | 4.1±0.5 |
| Epicatechin (EC) | 3.7±0.6 |
| Epigallocatechin (EGC) | 11.2±1.4 |
| Epicatechin gallate (ECG) | 3.8±0.6 |
| Epigallocatechin gallate (EGCG) | 18.7±1.2 |
| Epi Catechin(EG, EGC, ECG, EGCG) | 37.4±1.2 |

### [Example 2]

After drying green tea leaves (*Camellia sinensis,* Jeju O'Sulloc Farm), distilled water in an amount equivalent to 20 times the weight of the dried green tea leaves was added to the dried green tea leaves, extracted at 60 °C for 2 hours, and freeze-dried to prepare a green tea extract.

### [Comparative Example 1] Preparation of a coffee extract

A coffee extract was prepared by adding 50 % (v/v) alcohol in an amount of equivalent to 20 times the weight of the beans to dry coffee beans, extracting the mixture at 60 °C for 2 hours, and freeze-drying the extracted mixture.

### [Experimental Example 1] Inflammatory response in cells by microplastics

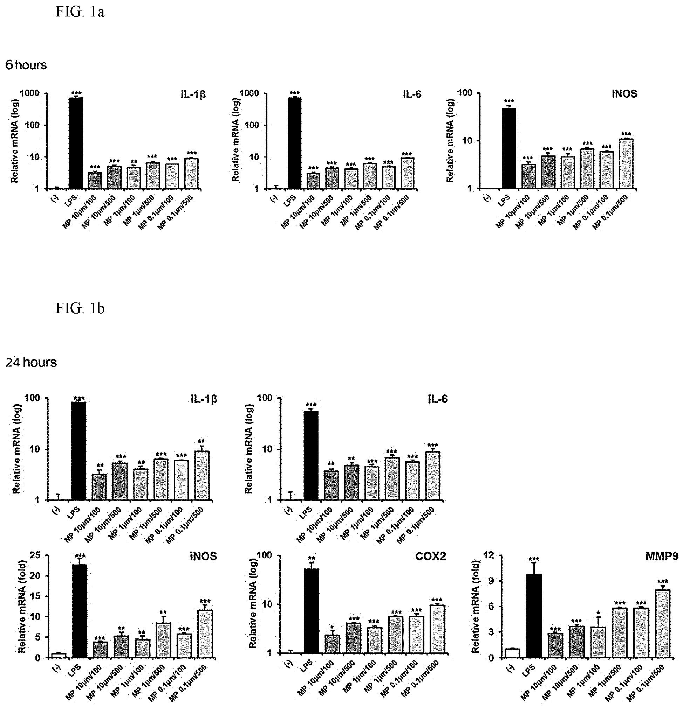

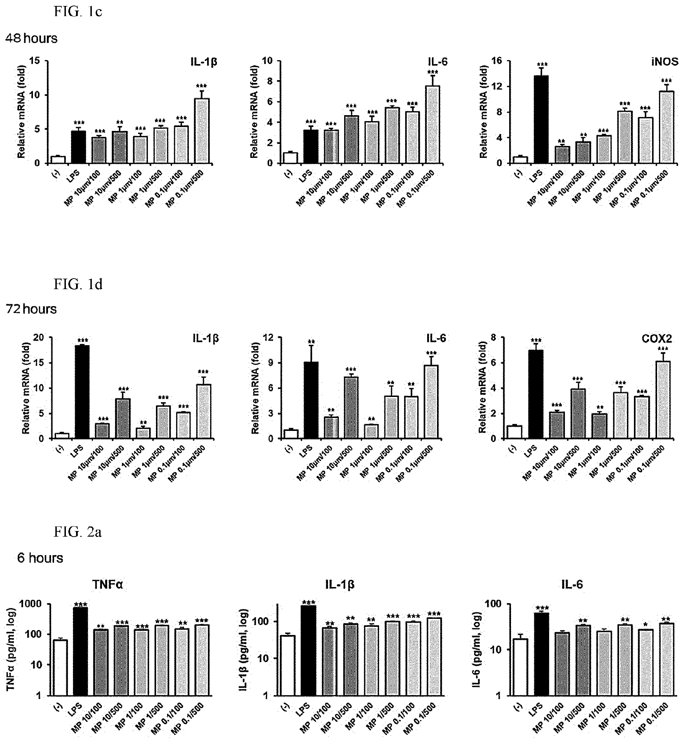

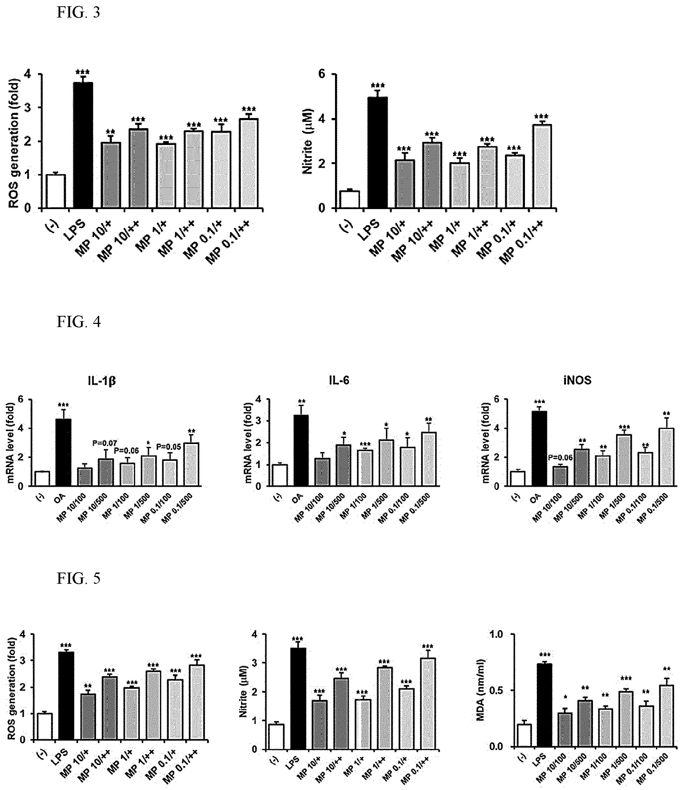

The microplastics are known to induce oxidative stress in cells. Since oxidative stress is closely related to a chronic inflammatory response, microplastics(MP) were treated in RAW 264.7 (ATCC) macrophage cell line to determine whether the microplastics can induce the chronic inflammatory responses in cells. The treated microplastics were polystyrene-based microparticles (Sigma Aldrich) with diameters of 10 µm, 1 µm, and 0.1 µm, respectively, the macrophage cell line was treated with each microplastic at concentrations of 100 µg/ml and 500 µg/ml for 6 to 72 hours. As a positive control for the inducement of the inflammatory response, 10 ng/ml of lipopolysaccharide (LPS; Sigma Aldrich), which was a component of the cell wall of Gram-negative bacteria and induced an acute inflammatory response, was used. At each time point (6 hours, 24 hours, 48 hours, 72 hours after the treatment of microplastic), RNAs were extracted from the cells using the MiniBEST Universal RNA Extraction kit (Takara Bio Inc.), and cDNAs were synthesized using a RevertAid 1st strand cDNA Synthesis kit (Thermo Fisher Scientific). The expression of genes related to the inflammatory response was determined with qPCR primers custom-made by Bioneer using a CFX96 thermocycler (Bio-Rad) and the results were shown in FIGS. 1a to 1d. In addition, the inflammatory factors secreted through the cell medium were measured using an ELISA kit (Abcam), and the results were shown in FIGS. 2a to 2d, and the oxidative stress involved in inducing an inflammatory response (the production of reactive oxygen species(ROS) and nitrite(NO)) was measured and shown in FIG. 3. Specifically, the ROS production was evaluated by treating the cells with 20 µM of H2-DCFDA (Invitrogen by Thermo Fisher Scientific) for 30 minutes, and measuring fluorescence values at wavelengths of Ex/Em 492/517 nm using a Tecan Infinite M200 Multiplate Reader (Tecan). The NO production was evaluated by measuring absorbance at 540 nm in Tecan Inifinite M200 using Griess reagent (Sigma Aldrich).

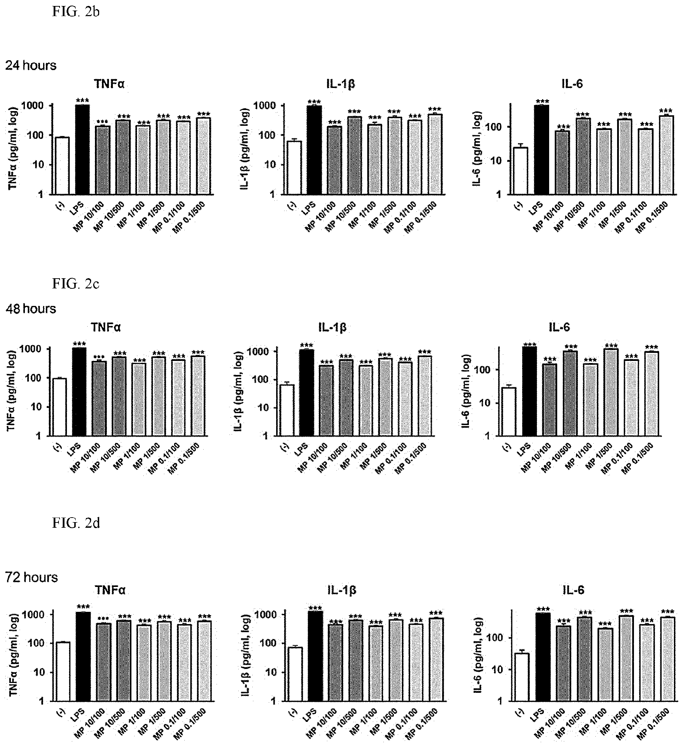

As shown in the results in FIGS. 1 and 2, it was found that the microplastics consistently induced the expression of inflammatory factors at a certain level. In addition, as shown in FIG. 3, it was found that the microplastics increased oxidative stress in the cells. Through these results, it was confirmed that the microplastics continuously induced the inflammatory response in the cell.

### [Experimental Example 2] Changes in lipid metabolism in cells by microplastics

Oxidative stress induces an inflammatory response and at the same time damages each tissue within the cell, causing cell metabolism disorder. Hepatocytes (HepG2; ATCC) were treated with the microplastics(MP) to examine whether the inflammatory response and oxidative stress caused by the microplastics change energy metabolism in cells. The treated microplastics were polystyrene-based microparticles (Sigma Aldrich) with diameters of 10 µm, 1 µm, and 0.1 µm, and the hepatocytes were treated with each microplastic at concentrations of 100 µg/ml and 500 µg/ml for 24 hours. As a positive control, oleic acid(OA) was used. Then, the expression of genes related to the inflammatory response and the degree of oxidative stress were measured in the same manner as in Experimental Example 1, and were shown in FIGS. 4 and 5, respectively.

As shown in FIGS. 4 and 5, it was confirmed that the microplastics induced the inflammatory reactions and the oxidative stress in hepatocytes, and lipid peroxides were significantly increased compared to a normal case. This is a phenomenon that occurs when lipid oxidation does not occur normally and oxidative stress occurs excessively, through this, it can be inferred that the microplastics cause abnormal lipid metabolism in cells.

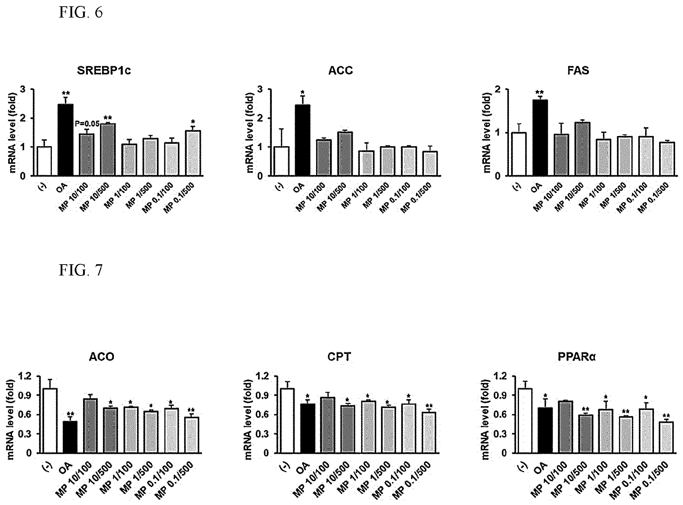

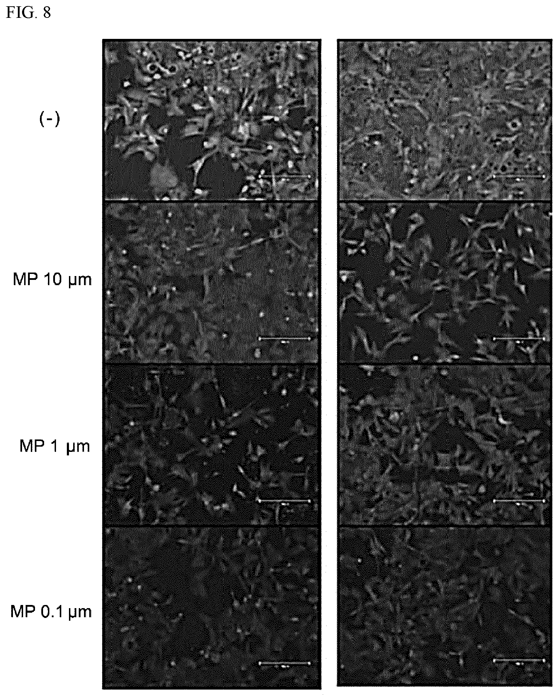

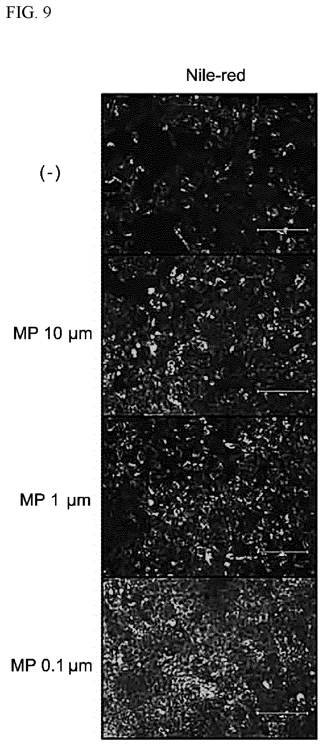

Accordingly, the expression of genes related to lipid metabolism was additionally measured in the microplastic-treated hepatocytes, and the results were shown in FIGS. 6 and 7. Specifically, RNAs were extracted from the hepatocytes using Takara MiniBEST Universal RNA extraction kit (Takara Bio), and cDNAs were synthesized using RevertAid First Strand cDNA Synthesis Kit (Thermo Fisher Scientific). Then, the expression of genes related to lipid metabolism was measured using Bio-Rad's CFX96 qPCR thermocycler. In addition, the mitochondria in hepatocytes were stained with 100 nM of Mitotracker Green^{™} FM (Invitrogen by Thermo Fisher Scientific) for 30 minutes and observed using a GFP filter of an EVOS M5000 microscope (Thermo Fisher Scientific), and the result was shown in FIG. 8. The triglycerides accumulated in hepatocytes were stained with 30 nM of Nile-red (Sigma Aldrich) for 30 minutes and observed using a RFP filter of an EVOS M5000 microscope, and the result was shown in FIG. 9 .

As shown in FIG. 6, the microplastics increased the expression of sterol regulatory element binding protein 1c (SREBP1c; a transcription factor that induces mRNA expression of a protein that induces fatty acid synthesis), but did not significantly increase the expression of genes involved in actual fat synthesis and storage, such as an acetyl-CoA carboxylase (ACC; enzymes mediating the first step of fatty acid synthesis) and a fatty acid synthase (FAS; attaching one carbon to malonyl-CoA to form a fatty acid chain). On the other hand, as shown in FIG. 7, the microplastics reduced the expressions of an acyl-CoA oxidase (ACO; a protein that directly induces the oxidation of fatty acids), a carnitine-palmitoyl transferase (CPT; an enzyme that transports fatty acid-CoA into the mitochondria for combustion) and a peroxisome proliferator-activated Receptor α (PPARα; a transcription factor that converts energy metabolism into a catabolic pathway). In addition, the microplastics decreased the number of mitochondria in the cells as shown in FIG. 8, and, increased lipid accumulation in hepatocytes as shown in FIG. 9. Through these results, it was found that the microplastics induced energy metabolism imbalance by inhibiting the catabolic pathway in cells, and caused an imbalance in redox balance, thereby increasing the oxidative stress and lipid peroxides in cells.

### [Experimental Example 3] Evaluation of an effect of aggregating microplastics

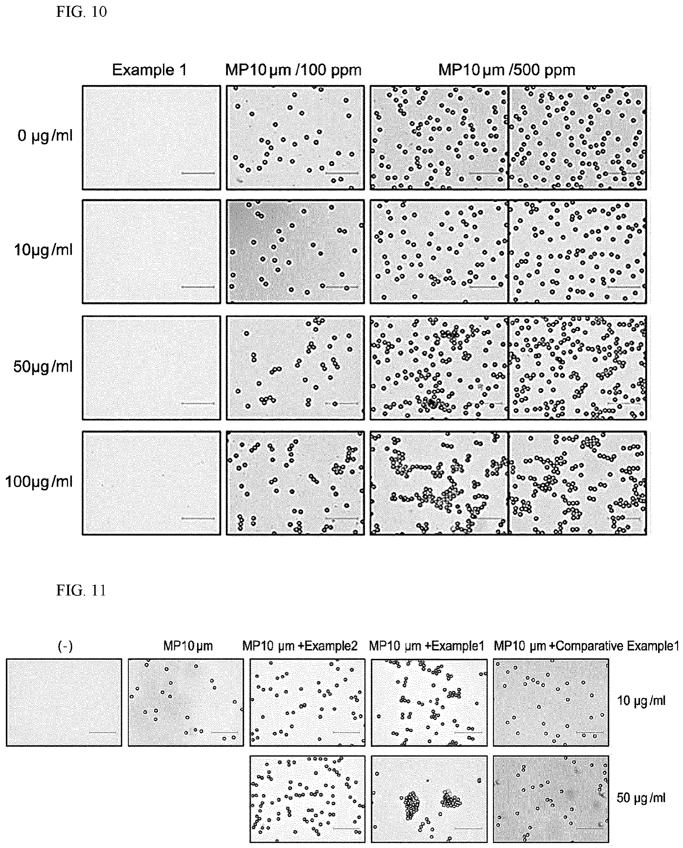

In order to determine whether the green tea extract induces the aggregation of the microplastics, the green tea extract of Example 1 was mixed with the microplastics(MP) at different concentrations (10 ug/ml, 50 ug/ml, 100 ug/ml) in PBS. The microplastics used were polystyrene-based microparticles with a diameter of 10 µm (Sigma Aldrich), and the concentrations of the microplastics were 100 ppm and 500 ppm. After mixing the green tea extract and the microplastics, the mixture was placed in a shaker for 2 hours to induce aggregation and observed under an EVOS M5000 microscope (Thermo Fisher Scientific), and the result was shown in FIG. 10.

As shown in FIG. 10, it was confirmed that the green tea extract of Example 1 induced the aggregation of the microplastics.

Each of Examples 1 and 2 and Comparative Example 1 was mixed with the microplastics by concentration (10 ug/ml, 50 ug/ml), and the degree of aggregation was observed and shown in FIG. 11.

As shown in FIG. 11, it was confirmed that microplastics were aggregated in the case of Examples 1 and 2, but in the case of Comparative Example 1, the microplastics were not aggregated.

### [Experimental Example 4] Evaluation of the efficacy of inhibiting the absorption of microplastics in the body

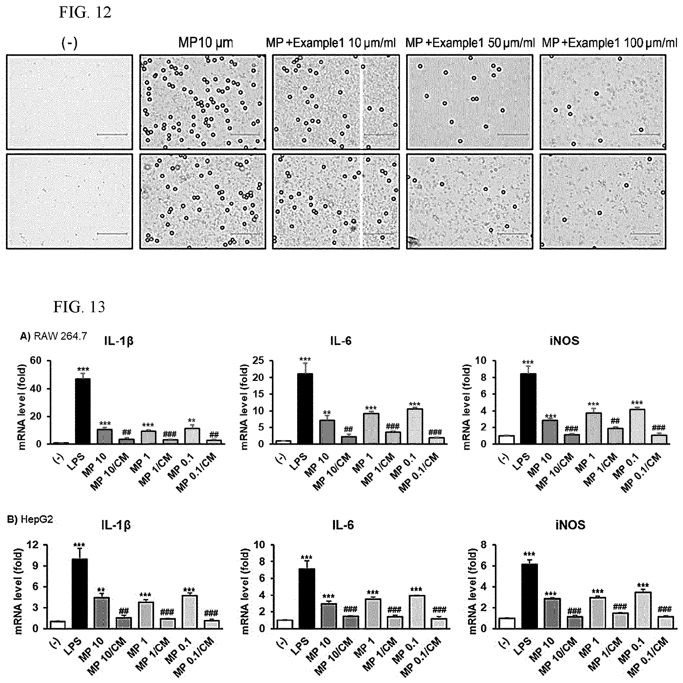

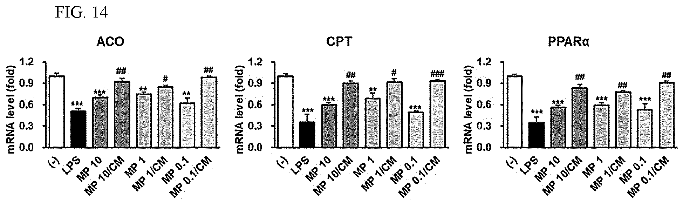

In order to examine whether the microplastic aggregation by the green tea extract affected the absorption of the microplastics in the body, a permeation experiment was conducted using CaCo₂ (ATCC) enterocytes, which were mainly used for unidirectional material transport experiments. Specifically, CaCo₂ cells were laid inside a transwell (Corning) and the medium was treated with the microplastics (diameter: 10 µm, Sigma Aldrich) and the green tea extract of Example 1 by concentration (10, 50, 100 ug/ml). Afterwards, the amount of microplastic that mitigated down the transwell through CaCo₂ was observed and shown in FIG. 12, and conditioned medium (CM; indicated microplastics + green tea extract 10 µg/ml) was treated to induce an inflammatory response in macrophages (RAW 264.7) and hepatocytes (HepG2). The degree of the inflammatory response was measured and shown in FIG. 13 . In addition, the expression levels of genes related to fatty acid oxidation (ACO, mCAD, CPT, PPARα) were measured and shown in FIG. 14.

As shown in FIG. 12, in the case that the green tea extract of Example 1 was treated, the amount of microplastics that migrated down the transwell was reduced, confirming that the green tea extract inhibited the absorption of the microplastics through the intestine. In addition, as shown in FIGS. 13 and 14, in the case that the absorption of the microplastics was inhibited by the green tea extract treatment, it was confirmed that inflammation in cells was reduced and energy metabolism disorder was alleviated.

### [Experimental Example 5] Evaluation of efficacy in alleviating abnormalities in cells caused by microplastics

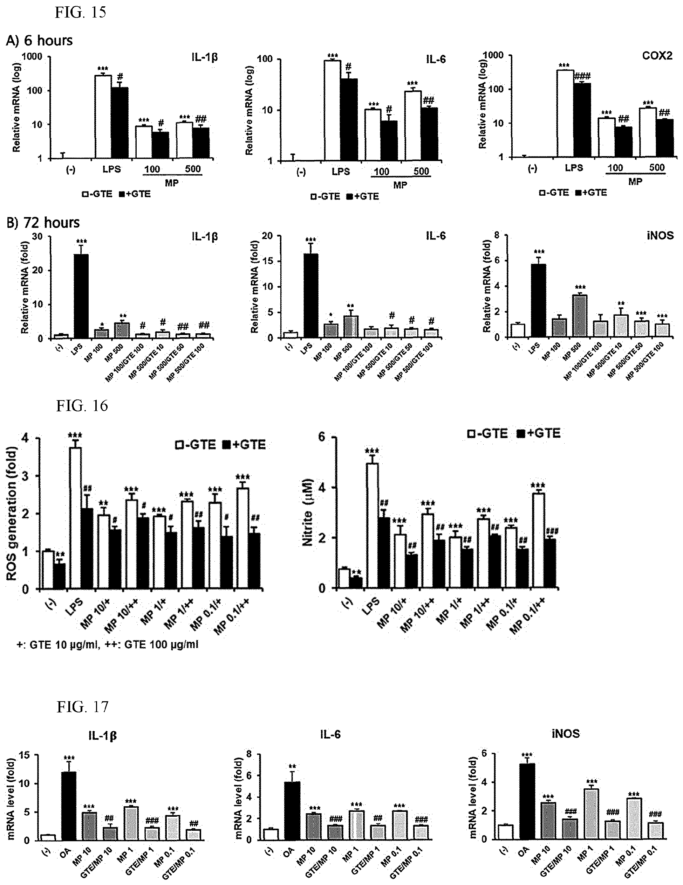

In Experimental Example 1, it was confirmed that the microplastics continuously induced inflammatory reactions in cells and increased oxidative stress. In order to determine whether the green tea extract could alleviate abnormalities in cells caused by the microplastics, macrophages were pretreated with the green tea extract of Example 1, and then the microplastics were treated in the same manner as in Experimental Example 1 to measure the degree of the expressions of genes related to the inflammatory response and the degree of oxidative stress, and the results were shown in FIGS. 15 and 16, respectively. GTE in FIGS. 15 and 16 refers to the green tea extract of Example 1.

As shown in FIGS. 15 and 16, the green tea extract reduced the inflammatory response caused by the microplastics, and inhibited the production of active oxygen and nitrogen compounds(NO) to alleviate the oxidative stress in cells.

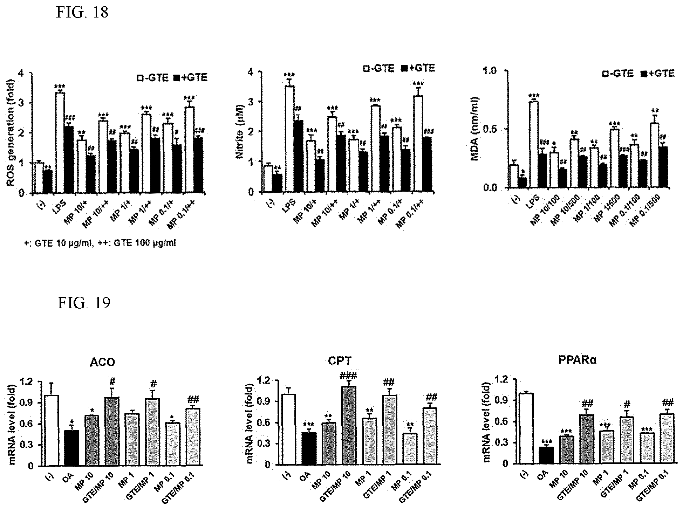

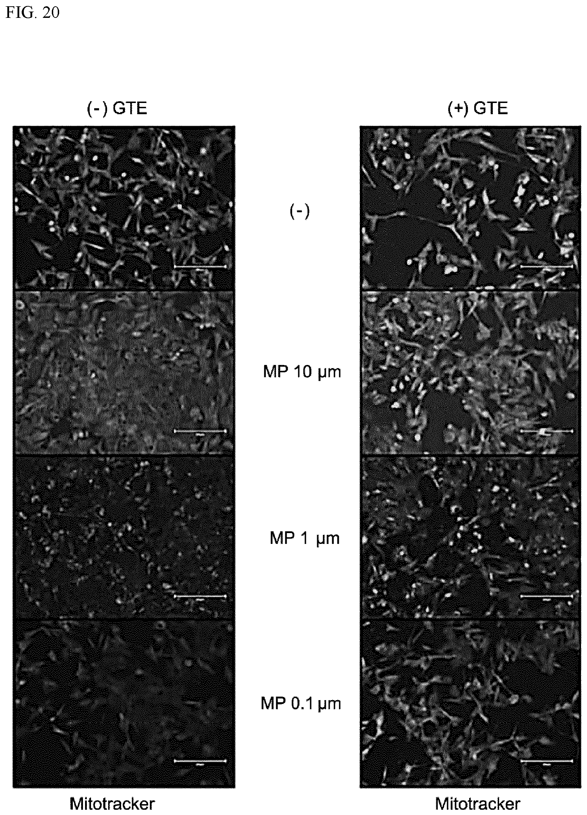

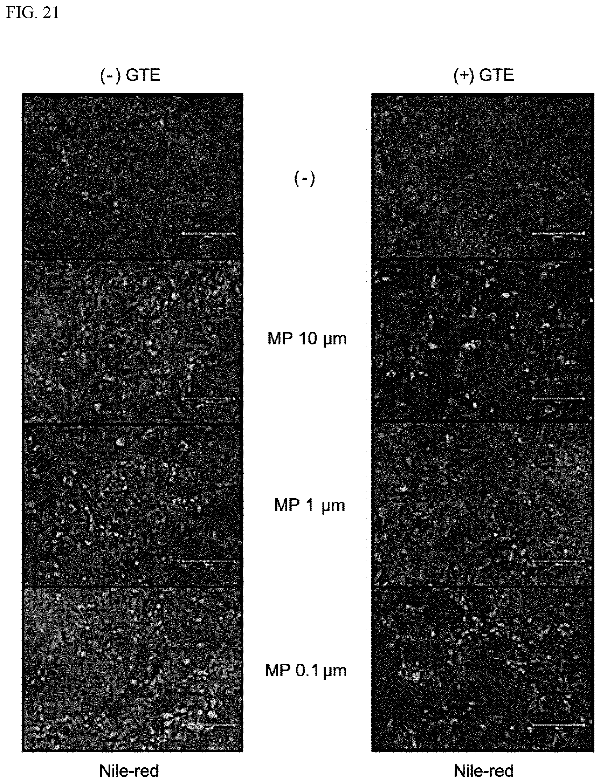

In addition, after the pretreatment of the green tea extract of Example 1 to hepatocytes, the microplastics were treated in the same manner as in Experimental Example 2 to measure the expression of genes related to the inflammatory response in hepatocytes (FIG. 17), the degree of oxidative stress (FIG. 18), the expression of genes related to lipid metabolism (FIG. 19), the number of mitochondria (FIG. 20), and the degree of the accumulation of lipids (FIG. 21), and the results were shown in FIGS. 17 to 21. GTE in FIGS. 17 to 21 refers to the green tea extract of Example 1.

As shown in FIGS. 17 and 18, the green tea extract reduced the inflammatory response and oxidative stress caused by the microplastics in hepatocytes, and as shown in FIG. 19, alleviated the lipid metabolism disorder. In addition, as shown in FIGS. 20 and 21, the green tea extract increased the number of mitochondria decreased by the microplastics and alleviated fat accumulation by the microplastics.

### [Formulation Example 1] Capsule

Tables were prepared by mixing 100 mg of the green tea extract (Example 1) according to examples of the disclosure, 400 mg of lactose, 400 mg of corn starch, and 2 mg of magnesium stearate, and then tableting the mixture according to a commonly employed method for preparing tablets. The specific composition was shown in Table 2 below.

**[Table 2]**

| Ingredient | Content (mg) |
|---|---|
| Green tea extract of Example 1 | 100 |
| Lactose | 400 |
| Corn starch | 400 |
| Magnesium stearate | 2 |

### [Formulation Example 2] Capsule

Capsules were prepared by mixing 150 mg of the green tea extract (Example 1) according to examples of the disclosure, 220 mg of soybean oil, 2 mg of palm oil, 8 mg of hydrogenated vegetable oil, 4 mg of yellow wax, and 6 mg of lecithin and filling the mixture in one capsule according to a commonly employed method for preparing capsules. The specific composition was shown in Table 3 below.

**[Table 3]**

| Ingredient | Content(mg) |
|---|---|
| Green tea extract of Example 1 | 150 |
| Soybean oil | 220 |
| Palm oil | 2 |
| Hydrogenated vegetable oil | 8 |
| Yellow was | 4 |
| Lecithin | 6 |

### [Formulation Example 3] Granule

Granules were prepared by mixing 80 mg of the green tea extract (Example 1) according to examples of the disclosure, 250 mg of anhydrous crystalline glucose and 550 mg of starch, forming the mixture into granules using a fluidized bed granulator, and then filling them into a pouch.

### [Formulation Example 4] Powder

A powder was prepared by mixing 200 mg of the green tea extract (Example 1) according to an embodiment of the disclosure, 500 mg of lactose, and 500 mg of cornstarch and filling the mixture in an airtight pouch.

### [Formulation Example 5] Health functional food

According to the composition shown in Table 4 below, health functional foods were prepared in a commonly employed manner.

**[Table 4]**

| Ingredient | Content |
|---|---|
| Green tea extract of Example 1 | 200 mg |
| Vitamin A acetate | 70 ug |
| Vitamin B1 | 0.13 mg |
| Vitamin B2 | 0.15 mg |
| Vitamin B6 | 0.5 mg |
| Vitamin B12 | 0.2 ug |
| Vitamin C | 10 mg |
| Biotin | 10 ug |
| Nicotinamide | 1.7 mg |
| Folic acid | 50 ug |
| Calcium Pantothenate | 0.5 mg |

### [Formulation Example 6] Drink

A drink was prepared by mixing 100 mg of the green tea extract (Example 1) according to an embodiment of the disclosure, 1000 mg of citric acid, 15 g of oligosaccharide, and 1 g of taurine, adding 190 ml of purified water and filling 200 ml in each bottle. After filling the mixture in the bottle, the bottle was sterilized for 4 to 5 seconds at 130° C to prepare the drink.

## Claims

1. A composition for use in preventing, alleviating or treating lipid metabolism disorder by reducing absorption of microplastics into the body, comprising a green tea extract as an active ingredient.

2. The composition for use of claim 1, wherein the green tea extract is an extract of water, an organic solvent, or a mixed solvent thereof.

3. The composition for use of any of claims 1 and 2, wherein the green tea extract comprises 30 to 45 % by weight of catechin based on a total weight of the green tea extract.

4. The composition for use of any of claims 1 to 3, wherein the green tea extract is administered at a dosage of 1 to 200 mg/kg/day.

5. The composition for use of any of claims 1 to 4, wherein the composition is administered to a subject in need of aggregating the microplastics.

6. The composition for use of any of claims 1 to 5, wherein the composition is administered to a subject in need of inhibiting inflammation caused by absorption of the microplastics.

7. The composition for use of any of claims 1 to 6, wherein the composition is administered to a subject in need of inhibiting oxidative stress in a cell caused by the microplastics.

8. The composition for use of any of claims 1 to 7, wherein the composition is administered orally.

9. The composition for use of any of claims 1 to 8, wherein the composition is a pharmaceutical composition.

10. Non-therapeutic use of a food composition for reducing absorption of microplastics into the body, comprising a green tea extract as an active ingredient.

11. The non-therapeutic use of claim 10, wherein the green tea extract is an extract of water, an organic solvent, or a mixed solvent thereof.

12. The non-therapeutic use of any of claims 10 and 11, wherein the green tea extract comprises 30 to 45 % by weight of catechin based on a total weight of the green tea extract.

13. The non-therapeutic use of any of claims 10 to 12, wherein the green tea extract is administered at a dosage of 1 to 200 mg/kg/day.

## Patentansprüche

1. Zusammensetzung zur Verwendung bei der Vorbeugung, Linderung oder Behandlung von Fettstoffwechselstörungen durch Reduzierung der Absorption von Mikroplastik im Körper, umfassend einen Grüntee-Extrakt als Wirkstoff.

2. Zusammensetzung zur Verwendung nach Anspruch 1, wobei der Grüntee-Extrakt ein Extrakt aus Wasser, einem organischen Lösungsmittel oder einem Gemisch davon ist.

3. Zusammensetzung zur Verwendung nach einem der Ansprüche 1 und 2, wobei der Grüntee-Extrakt 30 bis 45 Gew.-% Catechin, basierend auf einem Gesamtgewicht des Grüntee-Extrakts, umfasst.

4. Zusammensetzung zur Verwendung nach einem der Ansprüche 1 bis 3, wobei der Grüntee-Extrakt in einer Dosierung von 1 bis 200 mg/kg/Tag verabreicht wird.

5. Zusammensetzung zur Verwendung nach einem der Ansprüche 1 bis 4, wobei die Zusammensetzung einem Probanden verabreicht wird, bei dem das Aggregieren der Mikroplaste notwendig ist.

6. Zusammensetzung zur Verwendung nach einem der Ansprüche 1 bis 5, wobei die Zusammensetzung einem Probanden verabreicht wird, bei dem eine Entzündung, die durch die Absorption des Mikroplastiks verursacht wird, gehemmt werden muss.

7. Zusammensetzung zur Verwendung nach einem der Ansprüche 1 bis 6, wobei die Zusammensetzung einem Probanden verabreicht wird, bei dem der oxidative Stress in einer Zelle, der durch die Mikroplaste verursacht wird, gehemmt werden muss.

8. Zusammensetzung zur Verwendung nach einem der Ansprüche 1 bis 7, wobei die Zusammensetzung oral verabreicht wird.

9. Zusammensetzung zur Verwendung nach einem der Ansprüche 1 bis 8, wobei die Zusammensetzung eine pharmazeutische Zusammensetzung ist.

10. Nichttherapeutische Verwendung einer Nahrungsmittelzusammensetzung zur Reduzierung der Aufnahme von Mikroplastik in den Körper, die einen Grüntee-Extrakt als Wirkstoff umfasst.

11. Nichttherapeutische Verwendung nach Anspruch 10, wobei der Grüntee-Extrakt ein Extrakt aus Wasser, einem organischen Lösungsmittel oder einem Gemisch davon ist.

12. Nichttherapeutische Verwendung nach einem der Ansprüche 10 und 11, wobei der Grüntee-Extrakt 30 bis 45 Gew.-% Catechin, basierend auf einem Gesamtgewicht des Grüntee-Extrakts, umfasst.

13. Nichttherapeutische Verwendung nach einem der Ansprüche 10 bis 12, wobei der Grüntee-Extrakt in einer Dosierung von 1 bis 200 mg/kg/Tag verabreicht wird.

## Revendications

1. Composition pour utilisation dans la prévention, l'atténuation ou le traitement de troubles du métabolisme lipidique en réduisant l'absorption des microplastiques dans le corps, comprenant un extrait de thé vert comme principe actif.

2. Composition pour utilisation selon la revendication 1, dans laquelle l'extrait de thé vert est un extrait aqueux, d'un solvant organique ou d'un mélange solvant de ceux-ci.

3. Composition pour utilisation selon l'une des revendications 1 et 2, dans laquelle l'extrait de thé vert comprend 30 à 45% en poids de catéchine par rapport au poids total de l'extrait de thé vert.

4. Composition pour utilisation selon l'une des revendications 1 à 3, dans laquelle l'extrait de thé vert est administré à une dose de 1 à 200 mg/kg/jour.

5. Composition pour utilisation selon l'une des revendications 1 à 4, dans laquelle la composition est administrée à un sujet ayant besoin d'une agrégation des microplastiques.

6. Composition pour utilisation selon l'une des revendications 1 à 5, dans laquelle la composition est administrée à un sujet ayant besoin d'une inhibition de l'inflammation causée par l'absorption des microplastiques.

7. Composition pour utilisation selon l'une des revendications 1 à 6, dans laquelle la composition est administrée à un sujet ayant besoin d'une inhibition du stress oxydatif dans une cellule causé par les microplastiques.

8. Composition pour utilisation selon l'une des revendications 1 à 7, dans laquelle la composition est administrée par voie orale.

9. Composition une utilisation selon l'une des revendications 1 à 8, dans laquelle la composition est une composition pharmaceutique.

10. Utilisation non thérapeutique d'une composition alimentaire pour réduire l'absorption des microplastiques dans le corps, comprenant un extrait de thé vert comme principe actif.

11. Utilisation non thérapeutique selon la revendication 10, dans laquelle l'extrait de thé vert est un extrait aqueux, d'un solvant organique ou d'un mélange solvant de ceux-ci.

12. Utilisation non thérapeutique selon l'une des revendications 10 et 11, dans laquelle l'extrait de thé vert comprend 30 à 45% en poids de catéchine par rapport au poids total de l'extrait de thé vert.

13. Utilisation non thérapeutique selon l'une des revendications 10 à 12, dans laquelle l'extrait de thé vert est administré à une dose de 1 à 200 mg/kg/jour.
